# EUROPEAN PATENT SPECIFICATION

(11) **EP 2 061 807 B1**
(45) Date of publication and mention of the grant of the patent: **18.07.2012**
(21) Application number: 07766406.8
(22) Date of filing: 01.08.2007
(51) Int. Cl.: C07K 14/705

(54) **MHC oligomers, components thereof, and methods of making the same**
MHC-oligomere, Komponenten davon und Verfahren zu deren Herstellung
Oligomères MHC, composants de ceux-ci et procédés permettant de les fabriquer

(30) Priority: 03.08.2006 GB 0615546
(43) Date of publication of application: 27.05.2009
(73) Proprietor: ProImmune Limited, Oxford OX4 4GA (GB)
(72) Inventor: SCHWABE, Nikolai Franz Gregor, Oxford OX2 6BP (GB); TAN, Linda, Cheng-Choo, Oxford OX2 6BP (GB); GOUVEIA, Catherine Elizabeth, Witney OX28 3UD (GB); COX, Joanna, Oxford OX3 0AU (GB)
(74) Representative: Cremer & Cremer
(86) International application number: PCT/GB2007/002921
(87) International publication number: WO 2008/015425

(56) References cited:
- EP-A1- 1 844 072
- EP-A2- 0 665 289
- WO-A-96/26962
- WO-A-02/083906
- GB-A- 2 422 834
- US-A1- 2002 164 340
- YU YIK Y L ET AL: "Cutting edge: single-chain trimers of MHC class I molecules form stable structures that potently stimulate antigen-specific T cells and B cells" JOURNAL OF IMMUNOLOGY, THE WILLIAMS AND WILKINS CO. BALTIMORE, US, vol. 168, no. 7, 1 April 2002 (2002-04-01), pages 3145-3149, XP002433697 ISSN: 0022-1767
- GRAS-MASSE H: "Chemoselective ligation and antigen vectorization" BIOLOGICALS, ACADEMIC PRESS LTD., LONDON, GB, vol. 29, no. 3-4, 2001, pages 183-188, XP002225275 ISSN: 1045-1056
- HAFENSTEIN SUSAN L ET AL: "Genetic and functional analyses of the phiX174 DNA binding protein: The effects of substitutions for amino acid residues that spatially organize the two DNA binding domains." VIROLOGY, vol. 318, no. 1, 5 January 2004 (2004-01-05), pages 204-213, XP004816878 ISSN: 0042-6822
- HUMPHREYS R E ET AL: "Increasing the potency of MHC class II-presented epitopes by linkage to Ii-Key peptide" VACCINE, BUTTERWORTH SCIENTIFIC. GUILDFORD, GB, vol. 18, no. 24, June 2000 (2000-06), pages 2693-2697, XP004196901 ISSN: 0264-410X
- KOCHENDOERFER ET AL: "Site-specific polymer modification of therapeutic proteins" CURRENT OPINION IN CHEMICAL BIOLOGY, CURRENT BIOLOGY LTD, LONDON, GB, vol. 9, no. 6, December 2005 (2005-12), pages 555-560, XP005162588 ISSN: 1367-5931

## Description

The present invention relates to an MHC oligomer comprising a core structure and at least two functional MHC complexes having a peptide binding groove. Individual functional MHC complex monomers are oligomerised via their peptides bound in the peptide binding groove of the complex.

### Background of the Invention

Major Histocompatibility Complex (MHC) molecules, which are found on the cell surface in tissues, play an important role in presenting cellular antigens in the form of short linear peptides to T cells by interacting with T cell receptors (TCRs) present on the surface of T cells. They consist of alpha and beta chains, and a peptide bound in a groove formed by these chains when properly folded.

It has been established that isolated or recombinant forms of MHC-peptide molecules are useful for detecting, separating and manipulating T cells according to the specific peptide antigens these T cells recognise. It has also been understood that the interaction between MHC molecules and TCRs across cell surfaces is multimeric in nature and that the affinity of a single MHC molecule for a given TCR is generally quite low.

As a consequence, there has been an effort to develop multimeric forms of isolated or recombinant MHC-peptide molecules that have an increased functional avidity in order to make such molecules more useful in the applications described above.

European Patent Application EP 812 331 discloses a multimeric binding complex for labelling, detecting and separating mammalian T cells according to their antigen receptor specificity, the complex having the formula (α-β-P)ₙ, wherein (α-β-P) is an MHC peptide molecule, n is ≥ 2, α comprises an α chain of a MHC I or MHC II class molecule, β comprises a β chain of an MHC protein and P is a substantially homogeneous peptide antigen. The MHC peptide molecule is multimerised by biotinylating the C terminus of one of the α or β chain of the MHC molecule and coupling of MHC monomers to tetravalent streptavidin/avidin or by providing a chimeric protein of an MHC molecule which is modified at the C terminus of one of the α or β chain to comprise an epitope which is recognised by a corresponding antibody that serves as a multimerising entity. The document further teaches use of the MHC oligomers for detecting, labelling and separating specific T cells according to their TCR specificity.

WO 93/10220 discloses a chimeric MHC molecule, comprising the soluble part of an MHC molecule, which can be either class I or class II MHC fused to an immunoglobulin constant region. The MHC portion of the molecule comprises complementary α and/or β chains and a peptide is bound in the respective binding grooves of the MHC molecules. Due to the presence of the dimeric immunoglobulin scaffold these chimeric MHC-Ig molecules undergo self-assembly into a dimeric structure.

European Patent Application EP 665 289 discloses specific peptides, MHC molecules binding these peptides, and oligomers obtained by crosslinking of the respective MHC molecules having the specific peptide bound to them. Oligomerisation is achieved by using chemical crosslinking agents or by providing MHC chimeric proteins comprising an epitope, which is recognised by an immunoglobulin such as IgG or IgM. The MHC molecules may comprise a label and may be used for labelling, detecting, and separating T cells according to their specific receptor binding, and may eventually be employed in therapy of humans.

In an alternative embodiment EP 665 289 describes oligomeric MHC complexes, which are oligomerised by using an oligomerised form of the MHC binding peptides. The oligomeric peptides may be linked through chemical modifications on the peptide or the oligomeric peptide may already form a linear oligomer, i.e. one peptide chain having several MHC binding portions.

US 2002/0058787 discloses peptide oligomers comprising at least two MHC binding peptides joined by a flexible molecular linker. The MHC binding peptides can be MHC class I binding peptides or MHC class II binding peptides. Also disclosed is an oriented cloning method for producing such oligomers. The disclosed oligomers can be used, for example, in connection with methods for specifically activating or inhibiting the activation of CD4+ or CD8+ T cells. Such methods provide therapeutic approaches for the treatment of tumours, autoimmune disorders, allograft rejection and allergic reactions. These peptide oligomers would however not be well suited for forming isolated MHC-peptide multimers, since it is usually necessary to incubate soluble MHC complexes or chains thereof in the presence of a molar excess of peptide. This would lead to very incomplete oligomerisation of the complexes and a situation where multiple sections of MHC-binding peptide in the resulting complexes are not bound to an MHC-peptide complex, which in turn can lead to decreased specificity and higher background binding of such complexes.

When constructing MHC multimers it can be desirable to construct MHC peptide monomers first and then to multimerise these monomers by attaching them to a multivalent entity (for example, as described in US 5,635,363) or to one another. However the method described in US 5,635,363 requires an epitope or site for specific attachment of the monomers to a multivalent entity on the alpha or beta chain of the MHC peptide complex. In fact not many convenient ways exist to provide such a specific attachment site.

The simplest way may be to provide an antibody binding epitope at the C-terminal end of the MHC molecule and then multimerising the molecule via one or more antibodies that are specific for that epitope. The drawback of this technique is that monomeric antibody epitope interactions are typically not as strong as would be desirable and the resulting molecule could be quite large if it is multimerised in a two-step process, e.g. by binding epitope specific antibodies first and isotype specific antibodies second to the resulting MHC antibody complexes.

Any chemical site-specific modification of a polypeptide that has been produced by recombinant protein expression is difficult as most known targeted coupling methods are specific to one or several amino acids. As a consequence several amino acids are usually modified at the same time, including those of the antigen peptide bound in the MHC molecule after a monomeric complex is formed. This has an uncontrollable effect on the ability of the complex to bind to its complementary T cell receptor successfully. This holds the more so true for any random cross-linking process.

As an alternative, it has been suggested to oligomerize the MHC complexes using the biotin-streptavidin system (see e.g. US 5,635,363). This method requires the site directed enzymatic biotinylation of the MHC molecules near one of its carboxyl termini. An enzyme recognition peptide sequence of around 14 amino acids is fused to the C-terminus of one MHC peptide chain, which then allows for the complex to be biotinylated by using a biotinylating enzyme recognising this site. Biotinylation thus involves a substantial number of process steps, including several rounds of protein purification, and an enzymatic biotinylation reaction that can lead to significant loss of active MHC complexes. Further, controlling the biotinylation efficiency of monomeric MHC subunits and quality of the final multimeric product is difficult. For example, where a specific MHC complex comprising homogeneous peptides is to be synthesized and the synthesis yield is very low, protein losses in the biotinylation reaction, and lower than 100% biotinylation efficiency can drive the yield of the finished product below an acceptable level.

This methodology also limits the multimerisation method for the biotinylated complex to binding it to avidin family proteins, such as streptavidin, which tetramerises it or to cross-linked variants of such proteins. With cross-linked avidin family protein variants it is however difficult to control the valency of the complexes accurately. In situations where such a tetrameric or non-uniform valency multimer is not desirable or the use of streptavidin or molecules related to streptavidin is unwanted this methods also has serious limitations.

As noted above, a monomeric MHC peptide complex, comprising a peptide bound in the MHC binding groove, has only a low affinity for binding to its putative TCR. Further, MHC-peptide multimers must have a certain quaternary structure which promotes and maintains binding of the complex and reduces its dissociation from the TCR. As discussed above, Strominger et al. in US 2002/0058787 disclose oligomeric MHC binding peptides separated by linkers without any secondary structure. Here binding of these peptides to MHC complexes only form a suitable quaternary structure while the MHC complexes remain anchored in the cell surface *in vivo*.

It is the object of the invention to provide an improvement over the prior art by providing MHC oligomers that are improved regarding their capability of binding with the TCR, particularly the MHC oligomers having well controllable predetermined valency and advantageous steric conformation. It is further an object of the invention to provide an oligomer, which can be made with reasonable yields, and in superior purity.

### Summary of the Invention.

To overcome the abovementioned and other disadvantages of the prior art and to solve the above objects one core objective of the invention is to create MHC oligomers that connect monomeric MHC peptide complexes at their peptides via linking segments to a core structure through which the MHC peptide complexes are oligomerised. The linking segments have the structure and characteristics as described hereinafter in order to provide an improved steric conformation for these MHC oligomers to bind to the T cell receptors of antigen specific T cells.

There is provided an MHC oligomer comprising at least two functional MHC complexes having a peptide binding groove, each MHC complex having a peptide bound in the peptide binding groove of the MHC complex, further comprising a linking segment that is connected at its first end with the peptide and at its second end with a core structure, through which core structure oligomerisation occurs, wherein the linking segment is rigid and extended, wherein the linking segment has in its native conformation a length of more than 10 Angstrom, and wherein the linking segment is not a random coil.

The linking segment has an extended and rigid structure. Preferably, the linking segment is in its native conformation at least 20 Angstrom long and more preferably it is at least 30 Angstroms long. It further has preferably a regular secondary structure with phi angles typically varying by no more than 90 degrees and psi angles typically varying by no more than 45 degrees each. According to a further aspect, the linking segment has an extended and rigid structure with a ratio of length to molecular weight of the linking segment of 15 Angstrom per kilo Dalton or more.

In one embodiment, the linking segment is located between the recognition site and the first segment. The recognition site is in one form a modification which allows oligomerisation of at least two MHC binding polymers or linking through a core structure. In one aspect the linking segment is located between the recognition site and the first segment and the peptide is separated from the linking segment by a first flexible linker. In a further aspect the linking segment is located between the recognition site and the first segment and the peptide is separated from the linking segment by a first flexible linker and the linking segment is separated from the recognition site by a second flexible linker. It is preferred that the first flexible linker and/or the second flexible linker are significantly shorter than the linking segment.

### Brief Description of the Drawings

Fig. 1 shows schematically (a) first segment, (b) a second segment, and (c) an MHC binding polymer according to the invention;
Fig. 2 shows a class II MHC oligomer;
Fig. 3 shows a branched peptide suitable for multimerising MHC complex monomers at their binding peptide;
Fig. 4 shows a cyclic oligonucleotide, suitable for multimerising MHC complex monomers at their binding peptide; and
Fig. 5 shows modified peptides that enable forming multimeric MHC complexes without a separate multivalent entity.

### Detailed Description of the Invention

The MHC binding polymer described herein comprises a first segment and a second segment, wherein the first segment comprises a peptide capable of binding in a binding groove of an MHC molecule, and wherein the second segment comprises a linking segment and a recognition site for coupling the MHC binding polymer with a specific partner, wherein the linking segment is a polymer with a length of at least 10 Angstrom in its native conformation. The linking segment can provide space for binding, firstly, between an MHC-peptide complexes and the TCR, and, second, of the MHC-peptide complexes in an oligomers of the invention, e.g. as tetramer or pentamer, or multimer of other well defined valency. Preferably, the linking segment has at least 20 carbon atoms along its polymeric backbone. Further, preferably, at least a portion of the MHC binding polymer is synthetic.

Native conformation herein means the conformation that the second segment or linking segment assumes under conditions that do not perturb the integrity of functional MHC peptide complexes. The length of a polypeptide in its native conformation will typically be shorter than the length that it can assume in a maximally extended conformation.

The linking segment of the MHC binding polymer and the linking polymer has an extended and rigid structure. It is preferably a linear polymer molecule. However, this does not exclude the presence of short side chains as long as the extended nature is maintained. By extended is meant that the linking segment is longer than wide. The molecule's length axis is longer than the middle diameter of the linking segment, preferably at least double the length. Thus, it is a rather long but relatively thin molecule. To describe the requirement of being extended further ideally when the linker segment is encased fully in an ellipsoid and the volume of such ellipsoid is minimized while still fully encasing the linker segment then extended should mean that the shortest axis of the ellipsoid is less than half the length of the longest axis of the ellipsoid. Rigid is to be understood in contrast to flexible, meaning that the linking segment only has limited internal degrees of freedom, i.e. is fixed with relation to intra molecular movements, especially rotational, translational, or hinging movements.

The purpose of this arrangement is to be able to use the first and second segments in linking MHC peptide complexes to form oligomers thereof. The purpose of the rigid and extended linking segment in several aspects of the invention, is to provide a substantially fixed arm length to the final MHC oligomer, which defines both an appropriate spacing between the different MHC molecules and reduces the translational degrees of freedom between two MHC or more molecules in the same oligomer. This has the effect of increasing the avidity of binding between the MHC oligomer and the respective T cell receptors (TCRs). As mentioned, the linking segment is extended and rigid. The extended form should be configured so that the required spacing between the MHC-peptide complexes and the TCRs can be achieved without the linking segment causing too much steric perturbation. In one embodiment rigid hydrophilic alpha helices are used for these purposes, i.e. helices that under physiological conditions do not aggregate, but that form a stable conformation that is not a random coil. Typically the hydrophilic alpha helix will be stable in solution on it's own and therefore not aggregate or assemble with other proteins in order to establish and maintain its secondary structure. In this context a polymer with a random coil would mean a polymer that does not have a well defined native conformation, since its bonds are too flexible allowing it to assume many different conformations. As an example polypeptides constructed of glycine serine repeats are often employed as flexible linkers, which however do form random coils.

According to one embodiment, the second segment of the MHC binding polymer or the linking polymer comprises in a sub-segment thereof a peptide with a hydrophilic alpha helical conformation. Moreover, the second segment comprises a sub-segment that is at least one third of the length of the second segment and which sub-segment comprises less than 30% glycine residues. In one embodiment, the second segment or linking segment does not comprise any beta sheet or beta turns in its structure. In a further aspect, the second segment comprises in a sub-segment thereof a sub-segment derived from the exposed DE alpha helical segment of a calmodulin-like family protein or functionally equivalent variants, derivatives, or fragments thereof. In one embodiment, the sub-segment derived from the exposed DE alpha helical segment of a calmodulin-like family protein is derived from troponin c. The DE alpha helix of calmodulin-like family proteins and that of troponin c are described, e.g. in Sundaralingam et al. (1985) PNAS 82: 7944-7947.

The linking segment which is applicable to several aspects and embodiments of the invention is a polymer with a length of at least 10 Angstrom in its native conformation. This length is approximately equivalent to a non-random coil polypeptide of 10 amino acids. In a typical amino acid chain of a peptide, 3 atoms per individual amino acid are connected with each other. From the N terminus to the C terminus this amounts to about 3,5 Angstrom per amino acid, that means more than 30 Angstrom minimum length. However, in an alpha helical conformation each amino acid ∓ only causes a rise in the alpha helix along its axis by a distance of of about 1,5 Angstrom. In one embodiment, the polymer has at least 30 atoms along its polymeric backbone.

The term "polymeric backbone" is defined as shortest path through the polymer, i.e. the linking segment. In one embodiment the linking segments are peptide or nucleic acid duplexes.

In one embodiment, the second segment has at least a sub-segment thereof, which is of a peptidic structure. Further, in one embodiment there is an MHC binding polymer, wherein the second segment comprises in a sub-segment thereof a portion that is not a random coil. Moreover, the second segment preferably has in its native conformation a length of more than 20 Angstrom and more preferably of more than 30 Angstrom.

The linking segment is in its native conformation at least 10 Angstrom long. It further has a regular secondary structure with phi angles typically varying by no more than 90 degrees and psi angles typically varying by no more than 45 degrees each. In one embodiment the length of the linking segment, e.g. in the form of an alpha helix, will be similar as the length of from the top of the peptide binding groove of the MHC molecule through to the beginning of its transmembrane domain measured in the native state of such molecules. This length is typically between 45 and 70 Angstroms. In one embodiment, the linking segment contains a polyproline alpha helix with more than 10 proline residues, preferably more than 15 proline residues and most preferably more than 20 proline residues, wherein any non-proline amino acids in the alpha helix are less than 30% and more preferably is less than 15%.

The terms phi and psi angles are well known in the art of polymer science where in a linear polymer of concatenated residues phi is the angle of rotation at the bond connecting two residues and psi is the fold angle at the bond connecting two residues. The maximum value range for phi is 0-360 degrees and the maximum value range for psi is 0-180 degrees. In a polypeptide the phi and psi angles describe the position of two connected amino acids in the polypeptide with respect to one another.

In one embodiment, the linking segment is located between the recognition site and the first segment. The recognition site is in one embodiment a modification which allows oligomerisation of at least two MHC binding polymers through a core structure. In one aspect the linking segment is located between the recognition site and the first segment and the peptide is separated from the linking segment by a first flexible linker. In a further aspect the linking segment is located between the recognition site and the first segment and the peptide is separated from the linking segment by a first flexible linker and the linking segment are separated from the recognition site by a second flexible linker. It is preferred that the first flexible linker and/or the second flexible linker are significantly shorter than the linking segment. The objective of the first and second flexible linkers is to provide enough rotational degrees of freedom so that the MHC molecules in the oligomer can orientate to bind T cell receptors optimally and to minimize steric hindrance between the MHC molecules and the linking segment and, e.g. the multivalent entity and the linking segment. The first and second flexible linkers combined should be long enough so that the linking segment is sufficiently separated from the MHC binding groove that it allows to orientate the MHC molecules sufficiently to bind to its putative TCR. In a further aspect, the above flexible linkers will be substantially shorter than the linking segment so that the function of the linking segment is maintained, namely providing a substantially fixed arm length to the final MHC oligomer. The flexible linkers should preferably do not re-introduce translational significant degrees of freedom between the individual MHC molecules of the MHC oligomer.

In one embodiment in the MHC binding polymers described herein first and second segments are connected with each other wherein the first segment is coupled to the second segment via a bond selected from the group consisting of disulphide, thioester, thioether, thiazolidine, oxazolidine, oxime, hydrazone and peptide.

An MHC binding polymer is provided, comprising a first segment and a second segment, wherein the first segment comprises a peptide capable of binding in a binding groove of an MHC molecule, and wherein the second segment comprises a linking segment, wherein the linking segment is a polymer having an extended and rigid structure with a length of at least 10 Angstrom in its native conformation. Preferred embodiments of the second segment and the linking segment include any features described hereinbefore.

An MHC oligomer described herein comprises at least two functional MHC complexes having a peptide binding groove, each MHC complex having a peptide bound in the peptide binding groove of the MHC complex, wherein each peptide is part of an MHC binding polymer as described before. The recognition site of the MHC binding polymer is a modification, which allows oligomerisation of at least two MHC binding polymers through a core structure. The MHC complexes are preferably derived from the extra-cellular part of an MHC class I complex or from the extra-cellular part of an MHC class II complex. It is preferred that the MHC oligomer does substantially not contain sections of peptide that are not bound to an MHC peptide complex. Here peptide means the peptide that binds to an MHC molecule. In one embodiment, at least two of the MHC peptide complexes in the oligomer are able to arrange in a conformation wherein the binding grooves of these two MHC-peptide complexes are separated by a distance of at least 40, preferably at least 50 and most preferably at least 60 Angstrom. Further it is preferred, that substantially none of the amino-acid side chains of an MHC binding portion of the peptide and/or of MHC alpha or beta chains in the MHC peptide complexes comprised in the MHC oligomer have been modified.

According to another aspect an MHC oligomer comprises at least two functional MHC complexes having a peptide binding groove, each MHC complex having a peptide bound in the peptide binding groove of the MHC complex. The oligomer further comprises a linking segment having a rigid and extended structure that is connected at its first end with the peptide and at its second end with a core structure, through which core structure oligomerisation occurs. The connection between the linking segment and the peptide and the linking segment and the core structure can be of any kind, e.g. covalent or non-covalent. Further, the said two connections may be identical or different. An example of this embodiment is a fusion protein wherein the linking segment is fused to the subunits of a multivalent protein, e.g. streptavidin. For example a fusion protein tetramer could be made, wherein the linking segment is fused to each of the sub-units of streptavidin, thus resulting in a tetravalent fusion protein. Such a fusion protein may be coupled to at least two peptides capable of binding or bound in an MHC binding groove, as is disclosed above or known in the art. The linking segment in this embodiment benefits from all of the features described hereinbefore in connection with the linking segment.

Further the linking polymer described herein can be used to construct the MHC oligomers of the invention. On the one hand the linking polymer can be coupled through one of its recognition sites to a specific binding partner provided the peptide of an MHC peptide complex. It can then be coupled to a multivalent entity through the other of its recognition sites on specific binding partners provided on the multivalent entity. Binding to the MHC complex and to the multivalent entity can be in any order.

In one embodiment the MHC oligomers of the present invention are oligmerised through the peptide, also designated MHC binding peptide, by oligomerising in a highly specific manner functional, peptide-containing monomeric MHC peptide complexes after they have been assembled through a modification provided on the MHC binding polymer. For oligomerisation the polymer is oligomerised in highly specific manner through a core structure. For that purpose the MHC binding polymers are either attached to a multivalent entity or the polymers self-assemble or oligomerise, thereby creating a core structure of the oligomer formed.

The term "core structure" as used herein is intended to designate any entity allowing a simultaneous binding of MHC binding polymers and hence the monomeric MHC polymer complexes. The core structure may either be provided by a separate multimeric entity, to which the polymers are attached. In the alternative, the polymers themselves by oligomerisation may create such core structure. This includes the oligomerisation domain of the polymers, to which the MHC binding portion is bound and from which it is pending. In other words, the modification of the MHC binding polymers allows them to self assemble or align, thereby creating a new core structure to which all polymers are bound simultaneously.

As used herein, the terms "multimerisation" or "oligomerisation" designate the phenomenon of creating an at least for a desired time stable complex comprising at least two functional MHC monomers. Both terms are considered exchangeable.

Based on the type of oligomerisation chosen the MHC oligomers of the present invention can have a well controllable predetermined valency and very high purity since the individual monomeric MHC-peptide complexes can be purified and assembled separately and multimerised subsequently. In addition, in some embodiments the invention has the advantage that monomeric MHC-peptide complexes can be synthesized first and, if necessary, modified using well known synthesis methods. Multimerisation can take place after monomer synthesis without requiring the monomers to be further modified at that time, thus avoiding additional protein loss at that stage. Further, modifications for the purpose of subsequent multimerisation are only required to be made on the small MHC peptide and/or the MHC binding polymer bound in the MHC peptide binding groove. This can, however, conveniently be achieved using, e.g. solid phase synthesis technology, which allows great flexibility for introducing site-specific modifications.

As a consequence, MHC oligomers formed according to the invention can be designed to have advantageous steric conformation as they are easily oriented in a planar configuration with all MHC peptide-binding faces in the complex facing T cell receptors on the surface of antigen-specific T cells. They can also be made with a precise and well controlled stoichiometry. In addition, the oligomers formed will be substantially free of peptide MHC-binding portions not bound to an MHC molecule.

Functional monomeric MHC complexes can be formed first. This may either be by refolding of the relevant MHC alpha and beta chains from inclusion body material in the presence of the modified peptide or MHC binding polymer of interest or by expression of native monomeric MHC peptide complexes in an eukaryotic expression system preferably in the presence of the modified peptide of interest. In both cases the refolding may also be followed by peptide exchange of with such modified peptide or MHC binding polymer of interest.

Oligomerisation of the MHC complexes can occur after functional monomeric complexes of MHC and MHC binding polymer have been formed through an appropriate reaction chemistry or mechanism enabled in part by the modification provided on the MHC binding polymer. Typically, this will be carried out under physiological buffer conditions that do not disrupt or alter the binding properties of the monomeric MHC peptide or MHC -MHC binding polymer complexes. Described herein is thus also a high yielding method, which retains the functionality of the oligomerised MHC complex monomers.

For the avoidance of doubt the reference to monomeric MHC complexes of the invention made herein does not exclude that such monomeric complexes are already pre-multimerised to a certain degree before further oligomerisation occurs at their binding polymer. E.g. monomeric MHC-polymer complexes may already be provided as MHC-Ig fusion dimers, such as described in WO 93/10220 before being oligomerised to become higher-valent MHC-peptide or MHC-MHC binding polymer oligomers. Hence monomeric MHC polymer complexes as used herein merely refers to the fact that functional monomeric sub-units comprising the MHC binding polymer of interest will in one embodiment be formed first before further oligomerisation occurs at the polymer. Preferably, however, the oligomeric MHC complexes of the invention will be generated by forming truly monomeric MHC complexes in the first instance.

In a further aspect the invention thus provides a MHC oligomer comprising at least two functional MHC complexes having a peptide binding groove, each MHC complex having a first segment bound via the peptide in the peptide binding groove of the MHC complex, each first segment being coupled with a second segment having a linking segment which is rigid and extended and a recognition site which recognition site has a modification allowing highly specific oligomerisation. The modification may in one embodiment be selected from the group consisting of an attachment site or an oligomerisation domain. Depending on the modification chosen, oligomerisation of the functional MHC complexes then occurs through (i) binding of each polymer to a multivalent entity at the specific attachment site provided on or attached to each polymer, or (ii) alignment of the polymers through the oligomerisation domain provided on or attached to each polymer. In any case the MHC complexes in the oligomer are connected to a core structure provided by the multivalent entity or by alignment of the polymers.

As used herein the term "highly specific" oligomerisation means a specificity and/or selectivity of forming the oligomer (by attachment or alignment), preferably under physiological conditions, in a predetermined manner that avoids any modification to be made in the oligomerisation reaction to the amino acids of the alpha or beta chain of the monomeric MHC-polymer complexes or the amino acids of the MHC binding portion of the MHC binding peptide. In one embodiment the selectivity is preferably greater than 10 and more preferably greater than 100 and most preferably greater than 1000.

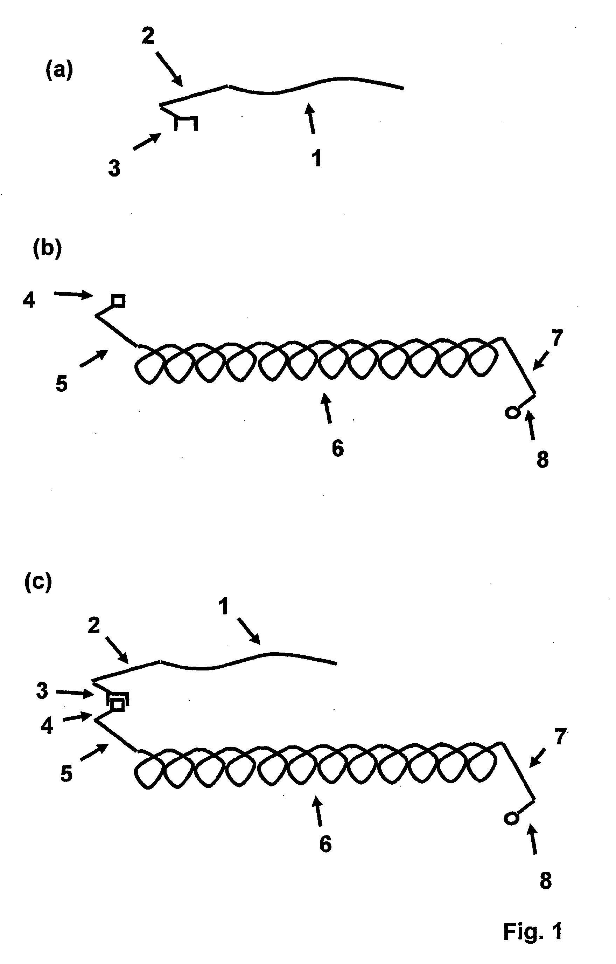

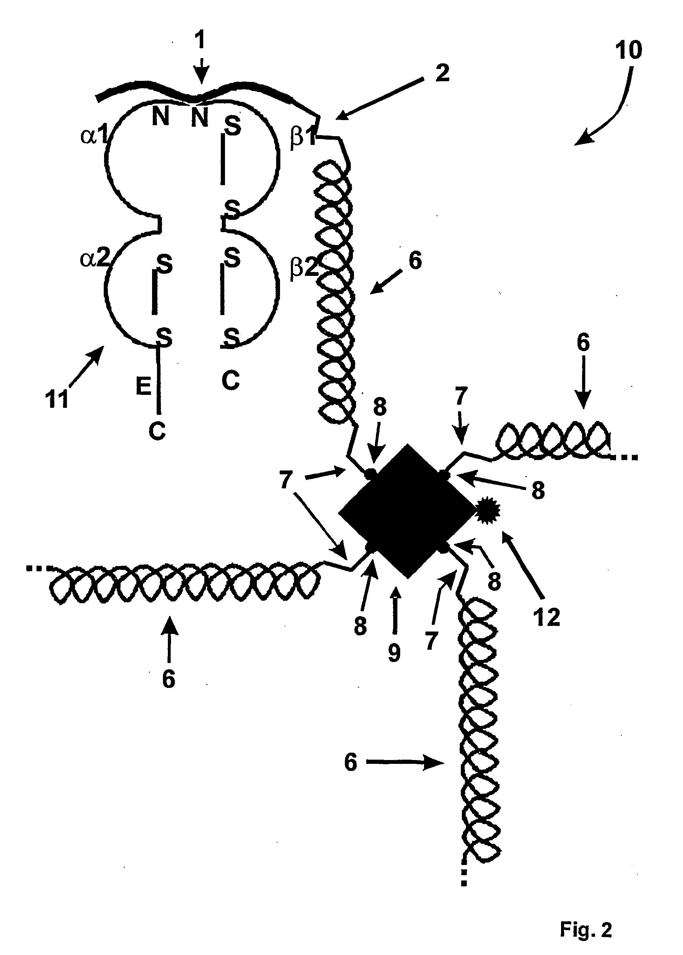

Figure 1 shows embodiments of the first segment (a), the second segment (b) and an MHC binding polymer (c) comprising the first and second segments of (a) and (b). As can be seen in Fig. 1(a), the first segment comprises a peptide 1, also known in the art as MHC binding peptide. The peptide 1 is capable of binding in the peptide binding groove of a functional MHC molecule (see Fig. 2). In the embodiment of Fig. 1, the peptide 1 is connected with a short flexible linker 2 such as a short glycine serine linker which comprises at its end a second specific binding partner 3. Figure 1(b) shows a second segment which comprises second recognition site 4 complementary to the binding partner 3 of the first segment. The second segment here is equivalent with the linking polymer of the invention. The second recognition site 4 is optionally connected to a short flexible linker 5, which is attached to a linking segment 6, which is again attached to a short flexible linker 7 which at its end comprises a first recognition site 8 suitable for binding to a first specific binding partner.

The recognition sites / binding partners may be members of a specific binding pair. Examples of specific binding pairs are hapten/antibody, epitope/antibody, ligand/receptor, substrate or substrate analogon/enzyme, cofactor or cofactor analogon/enzyme, nucleic acid/complementary nucleic acid, sugar/lectin, biotin/avidin family protein, such as avidin, streptavidin, neutravidin, Streptag® / Streptactin®. Where the recognition sites / binding partners are suitable for covalent ligation they are preferably groups for chemoselective ligation, i.e. a ligation process that does not modify any of the amino acid side chains in a native MHC peptide complex other than those of cysteine residues and more preferably none of these amino acid side chains are modified. In one aspect at least one of the first and second binding partners and first and second recognition sites is selected from the group consisting of a biotin moiety, a cysteine residue, an N-terminal cysteine residue, a maleimide group, an aldehyde group, a thioester group, a hydrazine group, a hydroxylamine group.

The objective of the flexible linkers 2, 5 and 7 is to provide enough rotational degrees of freedom so that the MHC molecules in the oligomer can orientate to bind T cell receptors optimally and to minimize steric hindrance between the MHC molecules and the linking segment 6 and, e.g. the multivalent entity and the linking segment 6. The linkers 2 and 5 combined should be long enough so that the segment 6 is sufficiently separated from the MHC binding groove so that it allows to orientate the MHC molecules sufficiently to bind to their putative TCR. The linkers 2, 5 and 7 will be substantially shorter than the linking segment 6 so that they do not contribute too much to re-introducing translational degrees of freedom between MHC molecules. At least the linker 5 is optionally and can be omitted. The same applies to the binding partner 3 and its complementary recognition 4. The peptide 1 can be coupled directly, in one embodiment via a short flexible linker 2, with the linking segment 6. Figure 2 shows such a configuration.

The MHC binding polymers peptides or linking polymers described herein can be synthesized by known methods for solid and solution phase organic synthesis methods. In one embodiment the entire MHC binding polymer may be substantially peptidic in nature. In this case the entire polymer can be synthesized by standard peptide synthesis methods. The recognition site can be introduced by chemical modification of one or more amino acids during synthesis or by the inclusion of pre-modified amino acids. Equally, if the MHC binding polymer is substantially a polypeptide it may be expressed in a recombinant expression system according to methods well known in the art. In this case the recognition site can be introduced, e.g. as a post translational modification or through an enzymatic reaction, e.g. such as the post translational modifications, such as incorporation of a biotin, a sugar as described in EP812331.

Figure 2 shows a class II MHC oligomer, in this case a tetramer, formed according to the invention. Four complexes 10 of MHC molecule and MHC binding polymer are oligomerised via a multivalent entity 9, here streptavidin. Of the four complexes 10 only one is shown fully, of the others only part of the linking segment 6 is shown. A monomeric class II MHC complex 11 has an antigenic peptide 1 bound in its groove. The figure shows the α1, α2 and β1, β2 domains of the MHC alpha and beta chains, respectively. The amino and carboxyl termini of the respective polypeptide chains are labelled N and C respectively. The location of disulphide bonds is indicated by S-S. The MHC alpha chain is shown to have a tag domain E. The antigenic or MHC binding portion of the peptide 1 is synthesized at one of its termini with a linker 2. This linker 2 allows a covalent site-specific attachment of the linking segment 6. The linking segment 6 is extended and rigid, e.g. an alpha helical polypeptide. A short flexible linker 7 is followed by a recognition site 8 which is a modification allowing oligomerisation of at least two MHC binding polymers through a core structure. In the embodiment of Fig. 2, the recognition site 8 is one member of a specific binding pair, here biotin. As can be seen, four MHC-polymer complexes are coupled via biotin to tetravalent streptavidin, forming a tetramer. The multivalent entity 9 can optionally bear a label 12, e.g. a fluorescent label.

For Class II MHC binding peptides where it is known that binding peptides can extend out of the MHC binding groove the modification may be made either on or near the N or the C terminus of the peptide.

Instead of a monomeric class II MHC complex a monomeric class I MHC complex that has an MHC binding polymer via an antigenic peptide bound in its groove, similar as the oligomer shown in Fig 2, may be used. In the case of a class I MHC molecule the linker 2 is preferably located at or near the C-terminus of the peptide, where the MHC binding peptide including the linker has more flexibility for overhanging the MHC binding groove than at the N-terminus.

Generally, oligomerisation can occur through recognition of the recognition site provided on the MHC binding polymer by a specific binding partner provided on a multivalent entity. According to one embodiment oligomerisation occurs through covalent binding of the MHC binding polymer to the multivalent entity. In this case the recognition site of the MHC binding polymer and the specific binding partner sites on the multivalent entity are moieties capable of creating a covalent bond with each other. In one embodiment, the covalent bond formed is of a type selected from the group consisting of disulphide, thioester, thioether, thiazolidine, oxazolidine, oxime, hydrazone and peptide.

In another embodiment oligomerisation occurs through non-covalent attachment of the MHC binding polymer to a multivalent entity. Here the recognition site will allow for non-covalent binding of the polymer to the multivalent entity at the complementary binding partner sites on the multivalent entity. The valency of the multivalent entity will be determined by the number specific binding partner sites and their individual valency that are provided on the multivalent entity and that are accessible for binding.

In one embodiment, if the attachment is non-covalent, the recognition site and the specific binding partner are selected from members of a specific binding pair. Suitable binding pairs of the necessary specificity are known in the art. Non-limiting examples of such binding pairs are hapten/antibody, epitope/antibody, legend/receptor, substrate or substrate analogue/ enzyme, cofactor or cofactor analogon/ enzyme, nucleic acid/complementary nucleic acid, sugar/lectin, biotin/avidin family protein, such as avidin, streptavidin, neutravidin, Streptag® / Streptactin®.

Streptag® is disclosed in US 5,506,121. It is a protein fusion tag having the amino sequence WXHPQFYZ where X, Y, Z are any amino acid, and preferably it is WSHPQFEK (single letter amino acid nomenclature).

Streptactin® is described in US 6,103,493 and refers to streptavidin muteins with the following characteristics: Polypeptide, selected from muteins of streptavidin, wherein it (a) contains at least one mutation in the region of the amino acid positions 44 to 53 with reference to the amino acid sequence of wild type streptavidin as set forth at SEQ ID NO: 17 in US 6,103,493 and (b) has a higher binding affinity than wild type-streptavidin for peptide ligands comprising the amino acid sequence WXHPQFYZ (SEQ ID NO: 16 in US 6,103,493) in which X between W and H represents an arbitrary amino acid and the two C terminal Y and Z either both denote G or the first denotes E and Z denotes R or K.

For example in case of the biotin/streptavidin binding pair, the biotin can be introduced into the MHC binding polymer at a pre-determined amino acid position through convenient chemical synthesis eliminating the need for cumbersome enzymatic biotinylation. Oligomerisation with streptavidin then results in a tetrameric MHC complex, as shown in Fig. 2.

Where the attachment of the MHC binding polymer to the multivalent entity occurs through covalent binding of the polymer to the multivalent entity the attachment site and the recognition site are complementary moieties capable of creating a covalent bond or coupling with each other, preferably under physiological conditions. Specific coupling approaches include, but are not limited to, the following types of covalent bonds: (a) oxime, (b) hydrazone, (c) thiazolidine, (d) oxazolidine, (e) thioether, (f) disulfide, (g) peptide, and (f) thioester. Several chemistries known in the art are available to form such types of bonds.

In one embodiment the moiety capable of creating a covalent bond or coupling is different from (i) a natural amino acid side chain and/or (ii) an amino acid carboxyl or amino terminal group and (iii) a combination of (i) and (ii).

In a more preferable embodiment any one of the more recently developed specific chemoselective ligation chemistries using thiol chemistry or carbonyl chemistry are applied. For further details reference should be made to J.P. Tam and Y.A. Lu in "Chemoselective and orthogonal ligation techniques" in chapter 11 of Weng C. Chan and Peter D. White Eds., "Fmoc solid phase peptide synthesis, A Practical Approach" Oxford University Press (2000), which is incorporated herein by reference. Commercially available specific cross-linking tools, which can be applied in the present invention, are e.g. available under the tradename HydraLinK® from EMD BioSciences, Inc., Darmstadt, Germany.

Characteristically such chemoselective ligation (i) uses unprotected peptide segments of the polymer and (ii) the reaction is performed in aqueous conditions. To achieve these chemistries, a reactive pair consisting of a nucleophile and an electrophile is placed on the second segment and the multivalent entity during their respective synthesis. Usually the nucleophile is a weak base, which has either a pKₐ significantly lower than the alpha- or epsilon-amines in natural amino acids or a nucleophilicity much stronger than such alpha-amines, so that the ligation can be selective in aqueous buffered solution at a pH of approximately 7. Cheomoselectivity is achieved when such mutually reactive groups are brought together in aqueous solution with the weak base as the sole nucleophile to react with the electrophile. Protection of other functional groups on the peptides involved therefore becomes unnecessary.

Generally it is preferable to use a chemoselective ligation chemistry that reacts complementary moieties provided as the recognition site on the MHC binding polymer and specific binding partner on the multivalent entity, whose reaction with one another can be carried out under environmental conditions such that none of the amino-acid side chains of naturally occurring amino acids will be modified in this reaction and that substantially do not ablate the functional integrity of native MHC-peptide complexes. In one embodiment, the moiety is useful in chemoselective ligation to the specific partner of the oligomer under conditions that preserve the functional integrity of the MHC-peptide complex formed by binding the MHC binding polymer to an MHC complex at its MHC binding peptide.

In one embodiment the recognition site or the specific binding partner will comprise an aldehyde group, which can be introduced into a polypeptide backbone as described in Tam and Lu *supra.* In one embodiment one or both of the recognition site and specific binding partners will comprise a group that can react with an aldehyde group to form (a) oxime, (b) hydrazone, (c) thiazolidine, (d) oxazolidine, (e) thioether, (f) thioester bond. In one embodiment the specific attachment site provided on the peptide will comprise an aldehyde group. It should be noted that all variants for the recognition site and its respective specific binding partner can be used in all locations and contexts in the present invention where such recognition sites and specific binding partners occur.

Examples for the reaction chemistries forming bonds of the type (a) to (d) above are as follows:
(a) Oxime 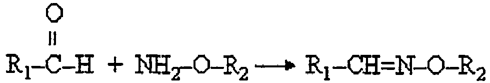
(b) Hydrazone 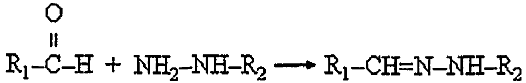
(c) Thiazolidine 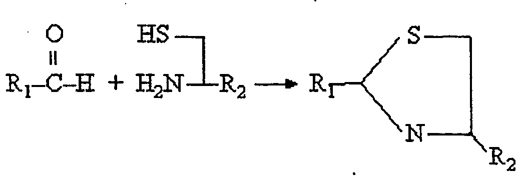
(d) Oxazolidine 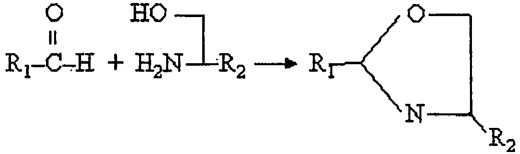
wherein R₁, R₂ in the context of the invention are non identical and selected from the group consisting of the MHC binding peptide, the MHC binding polymer the second or linking segment and the multivalent entity.

In each case the multivalent entity can be modified to incorporate a controlled number of copies of the recognition site. The system of complementary binding partners will have high specificity and selectivity. Each site may individually be monovalent or multivalent. In particular binding of the recognition site to the attachment site will be such that it occurs under conditions that do not impair the stability and activity of monomeric MHC polymer complexes, which means that it will typically be carried out under aqueous conditions and at near neutral pH.

It is also preferable that both sites are chosen such that the oligomerisation process does not modify any residue of the MHC binding peptide, MHC binding portion of the MHC binding polymer or the MHC alpha or beta chains.

Covalent coupling between the recognition site and the specific binding partner site can be preferable in some cases in order to maximize the stability of the oligomeric MHC complex. A simple approach of incorporating such reactive moieties is to incorporate a suitable modification at or near the amino or carboxy terminus of the multivalent entity and/or the MHC binding peptide or the MHC binding polymer during or after peptide synthesis using the protocols as discussed by Tam and Lu (*supra*).

Chemoselective non-amide ligation will be preferable for coupling, as it is site specific and the reaction is performed in aqueous conditions at a pH of 7 or close enough to 7 not to interfere with the structure of native MHC-peptide complexes.

It will be obvious to the skilled practitioner how to translate the above chemistries to a situation where the multivalent entity is not a polypeptide, but rather an oligonucleotide or another organic polymer.

Where a multivalent entity used for oligomerisation according to the present invention it can be any multivalent entity, as long as it does not unduly interfere with the T cell receptor binding of the MHC complex monomers. Valency of i.e. the number of specific binding partner sites or reactive moieties on the multivalent entity and their spacing will determine the degree of oligomerisation. For example, a tetravalent entity such as e.g. streptavidin will result in a tetramer. Much higher valencies are however possible. Preferably the valency of the entity will be in the range of 2 to 20, more preferably 4 to 10.

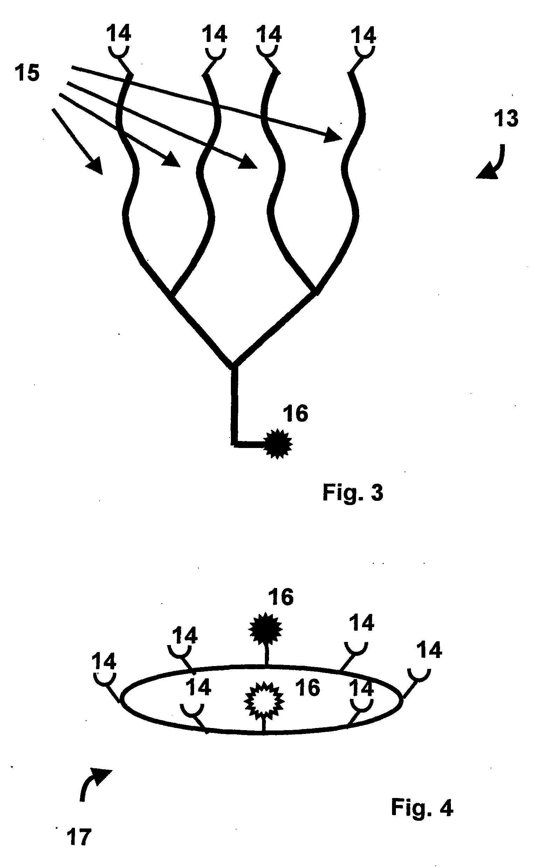

The multivalent entity can be a natural polymer or a derivative thereof such as a protein, a branched polypeptide (dendrimer) (see Fig. 3), a multimeric protein, a nucleic acid, a polysaccharide, such as dextran, starch, cellulose, hyaluronic acid, chitin, or alginic acid or a derivative of these polysaccharides, an oligonucleotide, a cyclic oligonucleotide (see Fig. 4); a synthetic polymer such as polypropyleneglycol, polyethyleneglycol (PEG), polyethylene oxide (PEO); a phospholipid membrane, such as a vesicle or a liposome, and an inorganic particle e.g. polystyrene or acrylic beads or magnetic beads. The specific partner may be provided by the multivalent entity e.g. by its backbone, or may be attached thereto.

In general the multivalent entity will itself be rigid and not introduce significant additional internal translational degrees of freedom to the monomeric MHC complexes in the oligomers. To the extent that the multivalent entity has flexible linking portions such as the dendrites in a dendrimer or in case it is an oligonucleotide, the structure will be either relatively rigid or to the extent that it is flexible, it will be short enough in order not to introduce significant additional degrees of freedom.

In one embodiment the multivalent entity is a natural polymer (such as a protein or multimeric protein e.g. streptavidin, avidin, an immunoglobulin, hyaluronic acid, cellulose) or synthetic polymer (e.g. polyacrylic acid, polystyrene, polylactic acid) and the recognition sites are provided by the backbone of the multivalent entity, or are attached thereto.

In another embodiment the multivalent entity is a branched polypeptide (a so-called dendrimer), as shown in Fig. 3. These dendrimers may for example be made according to the protocol as disclosed in Tam and Lu (*supra*). Several other methods for synthesizing branched polypeptides will be well known to the practitioner skilled in the art.

In one embodiment the branched polypeptide has specific binding partner sites or members of a specific binding pair incorporated at predetermined sites in two or more of its branches. Each branch of the peptide may have a desired length. Branching of the peptide may be effected by branching the peptide during synthesis on Lysine residues by known methods. In this manner the peptide is branched on a first lysine residue into two branches and further branched on further lysine residues to form a tetravalent entity thereafter. Other valencies, such as octamers, may be effected by including more or less branching steps. Odd valencies are also achievable by only partially branching the synthetic peptide.

Overall the chemical synthesis of the dendimer as the multivalent entity will allow controlling the nature and stoichiometry of oligomer very precisely. This in turn translates in higher precision in the various uses of the oligomer such as in T-cell labelling or detection. Equally the oligomerisation domains as described herein before can be used as multivalent entities in a situation where they are not pre-attached to their respective MHC binding polymers. Instead these oligomerisation domains can be used in their oligomeric form and be provided with specific binding partners as described herein so that the MHC binding polymers of the invention can be coupled thereto.

Fig. 3 shows a branched peptide or dendrimer suitable for multimerising MHC complex monomers at the MHC binding polymer according to the invention. The figure illustrates a polypeptide backbone 13 that is branched twice to yield a tetrameric structure. The dendrimer is synthesized to incorporate the complementary a specific binding partner site 14 on each terminal branch, which can bind to the specific recognition site 8 (see Figs. 1 and 2) under conditions that do not affect the stability of the MHC complexes, thus yielding a tetravalent entity. The figure further shows a fluorescent labelling moiety 15, which is incorporated in the unbranched portion of the dendrimer. In this example, for some aspects of the invention, the polymeric branches 15 of the multivalent entity may function as the linking segment in the oligomers of the invention. Here the linking segment will have any or all of the features of the linking segments described hereinbefore.

In an alternate embodiment the multivalent entity is an oligonucleotide or a cyclic oligonucleotide. The oligonucleotide will typically be 50 to 150 nucleic acids in length, but it may be as short as 3 nucleic acids. In one embodiment it is a cyclic oligonucleotide, which even more preferably is of less than 150 bases in length or circumference. This alternative embodiment is illustrated in Fig. 4, which shows the backbone of such a cyclic oligonucleotide 17. In analogy to Fig 3, here the oligonucleotide has been modified to include complementary recognition sites 14 on six different nucleotides, either during or after its synthesis, thus yielding a hexavalent entity. In addition the cyclic oligonucleotide also includes two fluorescent labelling moieties F.

According to the invention an alternative for oligomerisation the MHC oligomer is through alignment of the MHC binding polymers at their oligomerisation domains. With alignment we mean in the broadest sense any self-organized binding phenomenon between (macro)molecules, which result in at least a temporary association of two or more such (macro)molecules. Examples of this type are the hybridization of nucleic acids, the self assembly of two or more proteins (or protein domains) such as immunoglobulin Fc domains, coiled coil domains; self assembly of keratin fibres and similar and the covalent bonding of chemical moieties. The oligomerisation domains may actually form part of the peptidic backbone or may be attached thereto. They may be of peptide or nucleic acid character. Accordingly, in a first embodiment each polymer comprises a peptidic oligomerisation domain, which is in one embodiment is fused to the second segment. In an alternative embodiment each polymer has attached thereto an oligomerisation domain. In this case the oligomerisation domain may be selected from the group consisting of a peptidic oligomerisation domain and a nucleic acid.

The peptidic oligomerisation domain may be selected from the group consisting of an antibody constant domain, an enzyme monomer and an oligomerisation domain of an oligomer-forming coiled-coil protein. In one embodiment, it is an oligomerisation domain of an oligomer-forming coiled-coil protein selected from the group consisting of the protein families of collagens, C-type lectins and thrombospondin family proteins.

Examples for oligomer-forming coiled-coil proteins include various types of collagen, triple coiled-coil domains of C-type lectins, such as mannose binding protein (MBP); C1q, myosin, leucine zippers such those occurring in p53, GCN4, bacteriophage P22 Mnt repressor; and the trombospondin family proteins such as COMP. In one embodiment the oligomerisation domain is derived from the cartilage oligomeric matrix protein (COMP). Preferably, the oligomerisation domain is of the human version of COMP.

The number of MHC complexes (n) comprised on the MHC oligomer of the invention will in the case of coiled coil proteins typically depend on the type of oligomerisation domain the peptide is derived from and can in general be 2 or more, preferably n = 2 to 10, most preferably n = 3 or 5. If the modification of the peptides to be oligomerized is e.g. derived from the pentamerisation domain of the COMP this number will typically be five such that the oligomer will be a pentamer (n = 5), whereas in case these oligomerisation domains are derived from collagen this number will be three (n = 3).

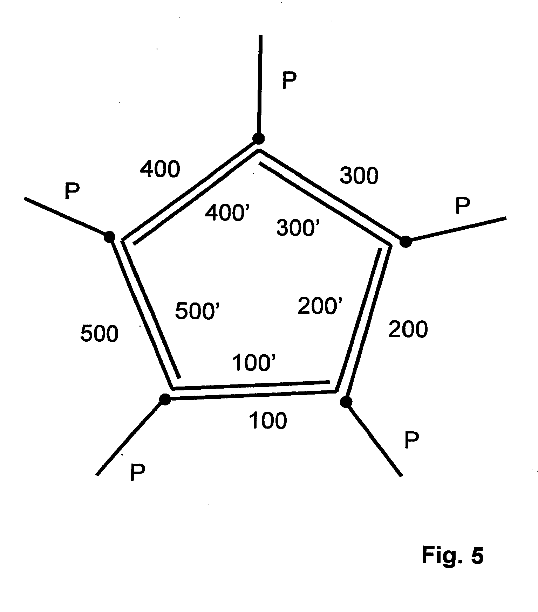

In one embodiment, illustrated in Fig. 5, each MHC binding polymer sequentially comprises two non-overlapping oligomerisation domains. The second oligomerisation domain of the n^{th} polymer in the oligomer then dimerises or aligns with the first oligomerisation domain of the (n+1)^{th} polymer in the oligomer. The second oligomerisation domain of the (n + 1)^{th} polymer in the oligomer then dimerises or aligns with the first oligomerisation domain of the (n+2)^{th} polymer in the oligomer and so on. Most preferably in this case, the second oligomerisation domain of the last polymer in the oligomer dimerises with the first oligomerisation domain of the first polymer in the oligomer to provide a cyclic structure. Although for this embodiment in principle any type of oligomerisation domain capable of dimerisation can be used, the first and second oligomerisation domains are preferably nucleic acids, oligomerisation occurring by hybridization thereof.

The functional monomeric MHC complexes to be oligomerised will usually be soluble isolated or recombinant MHC complexes that may be derived from MHC class I or class II complexes, preferably the extra-cellular part of an MHC class I complex or the extra-cellular part of an MHC class II complex. Each of these complexes consists of an alpha chain and a beta chain. The functional complex further comprises the MHC binding polymer having the peptide bound in the respective groove formed by the MHC conmplex's alpha and beta chains.

The MHC proteins to be used with the invention may be from any vertebrate species, e.g. primate species, particularly humans; rodents, including mice, rats, hamsters, and rabbits; equines, bovines, canines, felines; etc. Of particular interest are the human HLA proteins, and the murine H-2 proteins. Included in the HLA proteins are the class II subunits HLA-DPα, HLA-DPβ, HLA-DQα, HLA-DQβ, HLA-DRα and HLA-DRβ, and the class I proteins HLA-A, HLA-B, HLA-C, and β2 -microglobulin. Included in the murine H-2 subunits are the class I H-2K, H-2D, H-2L, and the class II I-Aα, I-Aβ, I-Eα and I-Eβ, and β2-microglobulin. Amino acid sequences of some representative MHC proteins are referenced in EP 812 331. Also included in the scope of this invention are non-classical examples such as HLA-E, HLA-F, HLA-G, Qa1, and CD1. The CD1 monomer may instead of the peptide have a lipid bound in its groove. The present invention is also applicable to the situation where a lipid instead of a peptide is bound and the skilled worker will be capable of translating the above oligomerisation protocols to this situation.

In a preferred embodiment, the MHC peptide chains correspond to the soluble form of the normally membrane-bound protein. For class I subunits, the soluble form is derived from the native form by deletion of the transmembrane and cytoplasmic domains. For class I proteins, the soluble form will include the α1, α2 and α3 domains of the α chain. For class II proteins the soluble form will include the α1 and α2 or β1 and β2 domains of the α chain or β chain, respectively.

Not more than about 10, usually not more than about 5, preferably none of the amino acids of the transmembrane domain will be included. The deletion may extend as much as about 10 amino acids into the α3 domain. Preferably none of the amino acids of the α3 domain will be deleted. The deletion will be such that it does not interfere with the ability of the α3 domain to fold into a functional disulfide bonded structure. The class Iβ chain, β2m, lacks a transmembrane domain in its native form, and does not have to be truncated. Generally, no class II subunits will be used in conjunction with class I subunits.

The above deletion is likewise applicable to class II subunits. It may extend as much as about 10 amino acids into the α2 or β2 domain, preferably none of the amino acids of the α2 or β2 domain will be deleted. The deletion will be such that it does not interfere with the ability of the α2 or β2 domain to fold into a functional disulfide bonded structure.

One may wish to introduce a small number of amino acids at the polypeptide termini, usually not more than 25, more usually not more than 20. The deletion or insertion of amino acids will usually be as a result of the requirements in cloning, e.g. as a consequence of providing for convenient restriction sites or the like, and to manage potential steric problems in the assembly of the molecules. In addition, one may wish to substitute one or more amino acids with a different amino acid for similar reasons, usually not substituting more than about five amino acids in any one domain.

In general, the oligomer may thus in one or more of the proteins or peptide chains comprised therein further comprise one or more additional domains such as one or more linkers, a tagging domain and a purification domain. The additional domain(s) may e.g. be provided on the multivalent entity or the peptide, but may also be present on the MHC alpha and/or beta chains.

For illustration purposes, the complex in Fig. 2 additionally has a protein tag E fused to one of their protein chains as a fusion polypeptide, which tag may allow further labelling, purification or attachment of these proteins. The tag E may be an epitope tag, or otherwise modified protein sequence. This tag may be separated from the polypeptide of the MHC alpha or beta chain by a polypeptide linker. In general this and any other linker will comprise not more than 25, preferably not more than 20 amino acids.

The protein tag E optionally included in the complexes as described above can be any domain which allows for labelling of the protein. In one embodiment the tagging domain includes a label. This label can be included in the domain itself such as an epitope recognised by an antibody or a light detectable or radioactive label. Preferably, the label is selected from the group consisting of fluorescent markers, such as such as FITC, phycobiliproteins, such as R- or B-phycoerythrin, allophycocyanin, Cy3, Cy5, Cy7, a luminescent marker, a radioactive label such as ¹²⁵I or ³²P, an enzyme such as horseradish peroxidase, or alkaline phosphatase e.g. alkaline shrimp phosphatase, an epitope, a lectin, biotin or streptavidin.

Where the label is itself a protein, the polypeptide chain of the protein used for labelling can be fused to the peptide to form a chimeric protein, preferably at its C terminus. For example a fluorescent protein such as a green fluorescent protein (GFP), or a subunit of a phycobiliprotein could be used in this chimeric protein. GFP chimeric protein technology is well known in the art. Chimeric proteins comprising a suitable domain from a phycobiliprotein are described, for example in WO 01/46395.

Alternatively labelling can be achieved by binding of a suitably labelled antibody, or antibody fragment, such as a labelled F(ab) fragment to a suitable epitope on the tag E.

The purification domain optionally to be included in the oligomers of the invention can be any domain assisting in purification of the protein of the invention e.g. by providing specific binding characteristics. Appropriate sequences are known to the skilled worker and can be applied as long as they do not interfere with the functional parts of the complexes. Preferably the purification domain is a hexahistidine sequence.

The functional monomeric MHC complexes to be oligomerised comprise the peptide or MHC binding polymer bound in their peptide binding groove. Where it is the MHC binding polymer it comprises at least the MHC binding portion and a separate portion attached to the second segment, the latter optionally via a short flexible linker, further the second segment bearing the modification in form of an attachment site or oligomerisation domain. The MHC binding portion of the peptide, excluding any linker, will be from about 6 to 14 amino acids in length for complexes with class I MHC proteins, and usually about 8 to 11 amino acids. It will be from about 6 to 35 amino acids in length for complexes with class II MHC proteins, usually from about 10 to 20 amino acids.

The peptides or MHC binding polymers in their MHC binding peptide portion may have a sequence derived from a wide variety of proteins. In many cases it will be desirable to use peptides, which act as T cell epitopes. The epitope sequences from a number of antigens are known in the art. Alternatively, the epitope sequence may be empirically determined by isolating and sequencing peptides bound to native MHC proteins, by synthesis of a series of putative antigenic peptides from the target sequence, then assaying for T cell reactivity to the different peptides, or by producing a series of MHC-peptide complexes with these peptides and quantification the T cell binding. Preparation of peptides, including peptide synthesis, identifying sequences, and identifying relevant minimal antigenic sequences is known in the art. In any case, the peptide comprised in the oligomeric MHC complex is preferably substantially homogeneous, meaning that preferably at least 80%, more preferably at least 90 % and most preferably at least 95% of the peptides are identical with regard to their MHC binding portion.

Typically a linking polypeptide sequence will be interposed between the sequence of the MHC binding portion of the peptide and the linking segment in the MHC binding polymer of the invention. Such linker may e.g. be a repeat of glycine residues, interspersed with prolines or serines, for flexibility and solubility. It will be appreciated that other linkers, which are flexible, have sufficient solubility and do not form significant secondary structure will also be suitable for this purpose. In general, polypeptide linkers of not more than 8 amino acids in length would be used. For non-peptidic linkers their length may be adjusted accordingly.

By provision of the linking segment, the recognition site on the second segment, e.g. the oligomerisation domain or attachment site, is at least far enough removed from the location of binding of the MHC-peptide complex to the T cell receptor (TCR) and the CD4, CD8 or other co-receptors such that it does not substantially interfere with the respective interaction of the MHC-peptide complex with the TCR.

An MHC oligomer according to the invention may also comprise a label. Such label may for example be selected from the group consisting of a light detectable label, a radioactive label, an enzyme, an epitope, a lectin, or biotin. Other labels have been listed above with regard to the tagging domain E. These may be used as well. If such entity is present, the label is in one embodiment provided on the multivalent entity or structure formed by alignment of the oligomerisation domains. The label may be incorporated during or after assembly of the oligomer or even during application or use of the same. Where the multivalent entity itself is formed by chemical synthesis the label may be chemically introduced at a predetermined location on the multivalent entity which will allow labelling of the oligomeric MHC complexes with precise stoichiometry. Where the label is a fluorescent label this will allow obtaining complexes with high uniform brightness and maximal signal to noise ratio.

The present description also pertains to a pharmaceutical or diagnostic composition comprising an MHC oligomer as defined above, optionally in combination with a pharmaceutically acceptable carrier.

Pharmaceutical compositions comprising the oligomers of the invention are useful for, e.g. parenteral administration, i.e. subcutaneously, intramuscularly or intravenously. In addition, a number of new drug delivery approaches are being developed. The pharmaceutical compositions of the present invention are suitable for administration using these new methods, as well.

The compositions for parenteral administration will commonly comprise a solution of the oligomer dissolved in an acceptable carrier, preferably an aqueous carrier. A variety of aqueous carriers can be used, e.g. buffered water, 0.4 % saline, 0.3 % glycine and the like. These solutions are sterile and generally free of particulate matter. These compositions may be sterilized by conventional, well-known sterilization techniques. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. The concentration of the oligomer in these formulations can vary widely, i.e. from less than about 1 pg/ml, usually at least about 0.1 mg/ml to as much as 10 - 100 mg/ml and will be selected primarily based on fluid volumes, viscosities, etc. in accordance with the particular mode of administration selected.

A typical pharmaceutical composition for intramuscular injection could be made up to contain 1 ml sterile buffered water, and 0.1 mg of oligomer protein. A typical composition for intravenous infusion could be made up to contain 250 ml of sterile Ringer's solution, and 10 mg of oligomer complex protein. Actual methods for preparing parenterally administrable compositions will be known or apparent to those skilled in the art.

The MHC oligomers of this invention can be lyophilized for storage and reconstituted in a suitable carrier prior to use. This technique has been shown to be effective and commonly used lyophilization and reconstitution techniques can be employed. It will be appreciated by those skilled in the art that lyophilization and reconstitution can lead to varying degrees of activity loss and that use levels may have to be adjusted to compensate.

The present description also relates to a method of labelling and/or detecting mammalian T cells according to the specificity of their antigen receptor, the method comprising
(i) combining an MHC oligomer according to the invention and a suspension or biological sample comprising T cells, and
(ii) detecting the presence of specific binding of said complex and the T cells.

The present description also relates to a method of separating mammalian T cells according to the specificity of their antigen receptor, the method comprising
(i) combining an MHC oligomer according to the invention and a suspension or biological sample comprising T cells, and
(ii) separating T cells bound to said complex from unbound cells.

Further the description also relates to a method for labelling and/or detecting and/or separating cells as described above wherein instead of T cells a type of lymphocyte is labelled, detected or separated according to a specific type of cell surface molecule that ∓ specifically binds to an MHC molecule. For example it is known that the human non-classical MHC molecule HLA-E can bind to Natural Killer (NK) cells via specific binding to the NK surface receptor CD94/NKG2 (D.S.J. Allan et al., J. Immunol. Meth. 268 (2002) 43-50). Similarly HLA-G and HLA-F was shown to stain ILT2 and ILT4 receptors on CD 14+ cells and HLA-F was also shown to stain a sub-population of CD 19+ (Allan *et al*., *supra*). The skilled worker will be capable of translating the methods described above to this situation.

In one embodiment, the monomeric functional MHC complexes are provided in step (ii) by refolding the MHC α and β chains in presence of the peptide as modified in step (i). In another embodiment the monomeric functional MHC peptide complexes are provided in step (ii) by peptide exchange for refolded functional MHC peptide complexes in the presence of the MHC binding polymer as modified in step (i).

The MHC α and β chains may e.g. be obtained from an eukaryotic or prokaryotic expression system and may optionally purified before refolding. Alternatively, the α and β chains or the folded complexes may be isolated from other sources. The method may, where desired or appropriate also comprise the step of peptide exchange for refolded functional MHC complexes. Providing the MHC binding polymer in step (i) will typically occur by solid phase synthesis as disclosed above. It may be modified either during or after said synthesis. Modification during synthesis will e.g. be by adding amino acids of peptide domains to the backbone or by incorporation of modified (e.g. thio-) amino acids. Modification after synthesis may e.g. be by attachment of a nucleic acid oligomerisation domain.

In Fig. 5 modified MHC binding polymers P are shown that enable forming multimeric MHC polymer complexes without a separate multivalent entity. The polymer will typically have a 10 amino acid glycine serine linker to provide sufficient spacing in the oligomer. The polymer is synthesized in five variants P(1) to P(5) to comprise five different oligonucleotides. Preferably P(1) to P(5) will comprise the same MHC binding portion in the peptide part of the MHC binding polymer.

Each attached oligonucleotide has two domains (100,200') (200,300'), (300,400'), (400,500'), and (500,100'), of which each one is complementary to one and only one other domain on another oligonucleotide. The fifth oligonucleotide has a second domain 1' that is complementary to the first domain 1 of the first oligonucleotide. Each domain pair may e.g. have an annealing temperature of about 20 °C. The cross-reactivity between non-complementary domains is to be minimized by known techniques of oligonucleotide design. The two domains on each oligonucleotide may be directly connected or have a non-annealing oligonucleotide linker, increasing the spacing between the domains. The linker may comprise a synthetic label, such as the labels described herein before. The coupled polypeptide-oligonucleotide may be synthesized in a single process or be synthesized separately with coupling carried out afterwards, wherein the peptide to oligonucleotide linkage can be achieved through chemoselective ligation of modified peptide to oligonucleotide as described above. The five oligo/peptide products are each purified to sufficient purity by HPLC.

The oligo/peptides are then annealed at 20 °C after heating to 30 °C for 5min. Oligo/peptide pentamers are then re-purified. Oligo/peptide pentamers are then refolded with denatured MHC alpha and beta chain according to known protocols. This will result in hetero-multimers of non-uniform valency being formed, as the refolding of MHC molecules is imperfect and given a suitable polymer excess it will mainly result in MHC-polymer monomers which have 4 additional polymers attached via oligo coupling (MHC-P₅). The resulting product is then concentrated and purified by gel-filtration chromatography to predominantly select MHC-P₅ monomers. The P5 interaction is then melted by heating the monomers to 30 °C for 5 minutes, resulting in MHC-P' monomers and free polymer. The polymer is then purified away rapidly on a PD10 column (Amersham Biosciences, Chalfont St. Giles, UK) at 30 °C. The resulting MHC-P monomers are then eluted from the column. As the individual P(1) to P(5) peptides will occur with roughly equal molar ratio in the purified product the individual MHC-P monomers can then be re-annealed to form MHC pentamers. Incorrectly annealed products are purified away in a second gel filtration step so that the purified product substantially only includes MHC pentamer.

In a final aspect the description relates to a method of forming an MHC binding polymer comprising a first segment and a second segment, wherein the first segment comprises a peptide capable of binding in a binding groove of an MHC molecule, and wherein the second segment comprises a linking segment and a recognition site for coupling the MHC binding polymer with a specific binding partner, the method comprising providing a linking polymer of the invention, the method further comprising providing a MHC binding polymer of the invention, and coupling the linking polymer to the MHC binding polymer by chemoselective ligation.

The MHC binding polymer formed by the above method may be purified by standard liquid chromatography methods.

## Claims

1. MHC oligomer comprising a core structure and at least two functional MHC complexes having a peptide binding groove, the MHC complexes having a peptide bound in the peptide binding groove of the MHC complex, the MHC complexes further comprising a linking segment that is connected at its first end with the peptide and at its second end with said core structure, through which core structure oligomerisation occurs, wherein the linking segment is rigid with limited internal degrees of freedom and extended, wherein the linking segment has in its native conformation a length of more than 10 Angstrom, and wherein the linking segment is not a random coil.

2. MHC oligomer of claim 1, wherein the linking segment has an extended and rigid structure with a ratio of length to molecular weight of the linking segment of 15 Angstrom per kilo Dalton or more.

3. MHC oligomer of any preceding claim, wherein the linking segment has in its native conformation a length of more than 20 Angstrom.

4. MHC oligomer of claim 3, wherein the linking segment has in its native conformation a length of more than 30 Angstrom.

5. MHC oligomer of any preceding claim, wherein the linking segment has at least a sub-segment thereof which is of a peptidic structure.

6. MHC oligomer of any preceding claim, wherein the linking segment has a regular secondary structure with phi angles typically varying by no more than 90 degrees and psi angles typically varying by no more than 45 degrees each.

7. MHC oligomer of any preceding claim, wherein the linking segment comprises in a sub-segment thereof a peptide with a hydrophilic alpha helical conformation.

8. MHC oligomer of any preceding claim, wherein the linking segment can be encased by a minimum volume encasing ellipsoid in which ellipsoid the shortest axis is half the length of the longest axis.

9. MHC oligomer of any preceding claim, wherein the core structure is a multivalent entity.

10. MHC oligomer of claim 9, wherein the multivalent entity is selected from the group consisting of a natural polymer or derivative thereof such as a protein, a branched polypeptide (dendrimer), PEG, PEO, a multimeric protein, a nucleic acid, a polysaccharide, such as dextran, starch, cellulose, hyaluronic acid, chitin, or alginic acid or a derivative of these polysaccharides, an oligonucleotide, a cyclic oligonucleotide; a synthetic polymer; a phospholipid membrane such as a vesicle or a liposome; and an inorganic particle.

11. MHC oligomer of any preceding claim, wherein at least 80% of the peptides are identical with regard to their MHC binding portion.

12. MHC oligomer of any preceding claim, wherein at least two of the MHC complexes in the oligomer are able to arrange in a conformation wherein the binding grooves of different ones of the MHC complexes in the oligomer are separated from one another by a distance of at least 40 Angstrom.

13. MHC oligomer of claim 12, wherein the distance is at least 50 Angstrom.

14. MHC oligomer of claim 12 or 13 wherein the distance is at least 60 Angstrom.

15. MHC oligomer of claim 9, wherein oligomerisation occurs through recognition of a recognition site provided on or attached to the peptide or the linking segment by a specific binding partner provided on the multivalent entity.

16. MHC oligomer of claim 15, wherein the recognition site and the specific binding partner are members of a specific binding pair and are selected from the group consisting of hapten/antibody, epitope/antibody, ligand/receptor, substrate or substrate analogon/enzyme, cofactor or cofactor analogon/enzyme, nucleic acid/complementary nucleic acid, sugar/lectin, biotin/avidin family protein, such as avidin, streptavidin, neutravidin, Streptag® / Streptactin®.

17. MHC oligomer of claim 15, wherein the oligomerisation occurs through covalent binding of the linking segment to the multivalent entity and wherein the recognition site on the linking segment and the specific binding partner on the multivalent entity are moieties capable of creating a covalent bond with each other.

18. MHC oligomer of any preceding claim, wherein oligomerisation occurs through alignment of the linking segment through an oligomerisation domain provided on each linking segment.

## Patentansprüche

1. MHC-Oligomer, umfassend eine Kernstruktur und wenigstens zwei funktionelle MHC-Komplexe mit einer Peptidbindungsgrube, wobei die MHC-Komplexe ein Peptid aufweisen, das in der Peptidbindungsgruppe des MHC-Komplexes gebunden ist, wobei die MHC-Komplexe weiter ein Verbindungssegment umfassen, welches an seinem ersten Ende mit dem Peptid verbunden ist und an seinem zweiten Ende mit dieser Kernstruktur verbunden ist, wobei durch die Kernstruktur die Oligomerisation geschieht, worin das Verbindungssegment rigide ist mit beschränkten inneren Freiheitsgraden und langgestreckt ist, worin das Verbindungssegment in seiner nativen Konformation eine Länge von mehr als 10 Angstrom hat, und worin das Verbindungssegment kein Zufallsknäuel ist.

2. MHC-Oligomer nach Patentanspruch 1, worin das Verbindungssegment eine langgestreckte und rigide Struktur hat mit einem Verhältnis von Länge zu Molekulargewicht des Verbindungssegments von 15 Angstrom pro kilo Dalton oder mehr.

3. MHC-Oligomer nach irgendeinem der vorstehenden Patentansprüche, worin das Verbindungssegment in seiner nativen Konformation eine Länge von mehr als 20 Angstrom hat.

4. MHC-Oligomer nach Patentanspruch 3, worin das Verbindungssegment in seiner nativen Konformation eine Länge von mehr als 30 Angstrom hat.

5. MHC-Oligomer nach irgendeinem der vorstehenden Patentansprüche, worin das Verbindungssegment wenigstens ein Untersegment aufweist, welches eine peptidische Struktur hat.

6. MHC-Oligomer nach irgendeinem der vorstehenden Patentansprüche, worin das Verbindungssegment eine regelmäßige Sekundärstruktur hat mit phi-Winkeln, die typischerweise nicht um mehr als 90 Grad variieren, und mit psi-Winkeln, die typischerweise jeweils nicht um mehr als 45 Grad variieren.

7. MHC-Oligomer nach irgendeinem der vorstehenden Patentansprüche, worin das Verbindungssegment in einem Untersegment davon ein Peptid mit einer hydrophilen alpha-helikalen Konformation umfasst.

8. MHC-Oligomer nach einem der vorstehenden Patentansprüche, worin das Verbindungssegment von einem ein Minimalvolumen umhüllendem Ellipsoid umhüllt werden kann, in welchem Ellipsoid die kürzeste Achse halb so lang ist wie die Länge der längsten Achse.

9. MHC-Oligomer nach irgendeinem der vorstehenden Patentansprüche, worin die Kernstruktur eine multivalente Einheit ist.

10. MHC-Oligomer nach Patentanspruch 9, worin die multivalente Einheit ausgewählt ist aus der Gruppe, bestehend aus einem natürlichen Polymer oder einem Derivat davon, wie z. B. einem Protein, einem verzweigten Polypeptid (Dendrimer), PEP, PEO, einem multimerischen Protein, einer Nukleinsäure, einem Polysacharid wie Dextran, Stärke, Zellulose, Hyaluronsäure, Chitin oder Algininsäure oder einem Derivat dieser Polysacharide, einem Oligonukleotid, einem zyklischen Oligonukleotid; einem synthetischen Polymer; einer Phospolipidmembran, wie ein Vesikel oder ein Liposom; und einem anorganischen Partikel.

11. MHC-Oligomer nach einem der vorstehenden Patentansprüche, worin wenigstens 80% der Peptide im Hinblick auf ihren MHC-Bindungsteil identisch sind.

12. MHC-Oligomer nach einem der vorstehenden Patentansprüche, worin wenigstens zwei der MHC-Komplexe in dem Oligomer in der Lage sind, sich in einer Konformation anzuordnen, worin die Bindungsgruben der unterschiedlichen MHC-Komplexe in dem Oligomer voneinander durch einen Abstand von wenigstens 40 Angstrom getrennt sind.

13. MHC-Oligomer nach Patentanspruch 12, worin der Abstand wenigstens 50 Angstrom beträgt.

14. MHC-Oligomer nach Patentanspruch 12 oder 13, worin der Abstand wenigstens 60 Angstrom beträgt.

15. MHC-Oligomer nach Patentanspruch 9, worin die Oligomerisation durch Erkennung einer Erkennungsstelle geschieht, die auf dem Peptid oder dem Verbindungssegment durch einen spezifischen Bindungspartner, der auf der multivalenten Einheit bereitgestellt wird, bereitgestellt wird oder daran angeheftet ist.

16. MHC-Oligomer nach Patentanspruch 15, worin die Erkennungsstelle und der spezifische Bindungspartner Mitglieder eines spezifischen Bindungspaares sind und ausgewählt sind aus der Gruppe, bestehend aus Hapten/Antikörper, Epitop/Antikörper, Ligand/Rezeptor, Substrat oder Substratanalogon/Enzym, Kofaktor oder Kofaktoranalogon/Enzym, Nukleinsäure/komplementäre Nukleinsäure, Zucker/Lektin, Biotin/Avidin-Familienprotein wie Avidin, Streptavidin, Neutravidin, Streptag®/Streptactin®.

17. MHC-Oligomer nach Patentanspruch 15, worin die Oligomerisation durch kovalentes Binden des Verbindungssegments an die multivalente Einheit geschieht und worin die Erkennungsstelle auf dem Verbindungssegment und der spezifische Bindungspartner auf der multivalenten Einheit Einheiten sind, die dazu in der Lage sind, eine kovalente Bindung miteinander zu schaffen.

18. MHC-Oligomer nach einem der vorstehenden Patentansprüche, worin die Oligomerisation durch Anordnen des Verbindungssegments durch eine Oligomerisationsdomäne, die auf jedem Verbindungssegment bereitgestellt wird, geschieht.

## Revendications

1. Oligomère CMH comprenant une structure de noyau et au moins deux complexes CMH fonctionnels ayant une rainure de liaison peptidique, les complexes CMH ayant un peptide attaché dans la rainure de liaison peptidique du complexe CMH, les complexes CMH comprenant de plus un segment de liaison qui est relié à sa première extrémité au peptide et à sa seconde extrémité à ladite structure de noyau, structure de noyau par laquelle a lieu l'oligomérisation, le segment de liaison étant rigide avec des degrés de liberté interne limités et étendu, le segment de liaison ayant, dans sa conformation naturelle, une longueur de plus de 10 Ångström et le segment de liaison n'étant pas une bobine aléatoire.

2. Oligomère CMH selon la revendication 1 dans lequel le segment de liaison a une structure étendue et rigide avec un rapport de longueur/poids moléculaire du segment de liaison de 15 Ångström par kilo Dalton ou plus.

3. Oligomère CMH selon l'une quelconque des revendications précédentes dans lequel le segment de liaison a, dans sa conformation naturelle, une longueur de plus de 20 Ångström.

4. Oligomère CMH selon la revendication 3 dans lequel le segment de liaison a, dans sa conformation naturelle, une longueur de plus de 30 Ångström.

5. Oligomère CMH selon l'une quelconque des revendications précédentes dans lequel le segment de liaison a au moins un sous-segment de celui-ci qui est de structure peptidique.

6. Oligomère CMH selon l'une quelconque des revendications précédentes dans lequel le segment de liaison a une structure secondaire régulière avec des angles phi qui varient typiquement de moins de 90 degrés et des angles psi qui varient typiquement de moins de 45 degrés chacun.

7. Oligomère CMH selon l'une quelconque des revendications précédentes dans lequel le segment de liaison comprend, dans un sous-segment de celui-ci, un peptide de conformation alpha-hélicoïdale hydrophile.

8. Oligomère CMH selon l'une quelconque des revendications précédentes dans lequel le segment de liaison peut être enveloppé par une enveloppe ellipsoïde de volume minimum dans lequel ellipsoïde l'axe le plus court est la moitié de la longueur du plus long axe.

9. Oligomère CMH selon l'une quelconque des revendications précédentes dans lequel la structure du noyau est une entité multivalente.

10. Oligomère CMH selon la revendication 9 dans lequel l'entité multivalente est sélectionnée dans le groupe comprenant un polymère naturel ou un dérivé de celui-ci tel qu'une protéine, un polypeptide ramifié (dendrimère), du PEG, du PEO, une protéine multimérique, un acide nucléique, un polysaccharide tel que le dextrane, l'amidon, la cellulose, l'acide hyaluronique, la chitine ou l'acide alginique ou un dérivé de ces polysaccharides, un oligonucléotide, un oligonucléotide cyclique ; un polymère synthétique ; une membrane phospholipide telle qu'une vésicule ou un liposome ; et une particule anorganique.

11. Oligomère CMH selon l'une quelconque des revendications précédentes dans lequel au moins 80% des peptides sont identiques pour ce qui est de leur portion de liaison CMH.

12. Oligomère CMH selon l'une quelconque des revendications précédentes dans lequel au moins deux des complexes CMH dans l'oligomère sont capables de s'arranger dans une conformation dans laquelle les rainures de liaison de différentes complexes CMH dans l'oligomère sont séparées l'une de l'autre par une distance d'au moins 40 Ångström.

13. Oligomère CMH selon la revendication 12 dans lequel la distance est d'au moins 50 Ångström.

14. Oligomère CMH selon la revendication 12 ou 13 dans lequel la distance est d'au moins 60 Ångström.

15. Oligomère CMH selon la revendication 9 dans lequel l'oligomérisation se fait par reconnaissance d'un site de reconnaissance prévu sur ou attaché au peptide ou au segment de liaison par un partenaire de liaison spécifique prévu sur l'entité multivalente.

16. Oligomère selon la revendication 15 dans lequel le site de reconnaissance et le partenaire de liaison spécifique sont membres d'une paire de liaison spécifique et sont sélectionnés dans le groupe comportant haptène/anticorps, épitope/anticorps, ligand/récepteur, substrat ou analogue du substrat/enzyme, cofacteur ou analogue du cofacteur/enzyme, acide nucléique/acide nucléique complémentaire, sucre/lectine, biotine/protéine de la famille avidine telle que l'avidine, la streptavidine, la neutravidine, Streptag® / Streptacin®.

17. Oligomère selon la revendication 15 dans lequel l'oligomérisation est effectuée par liaison covalente du segment de liaison à l'entité multivalente et dans lequel le site de reconnaissance sur le segment de liaison et le partenaire de liaison spécifique sur l'entité multivalente sont des moitiés capables de créer une liaison covalente l'une avec l'autre.

18. Oligomère CHM selon l'une quelconque des revendications précédentes dans lequel l'oligomérisation est effectuée par alignement du segment de liaison par un domaine d'oligomérisation prévu sur chaque segment de liaison.
